# EUROPEAN PATENT SPECIFICATION

(11) **EP 2 439 518 B1**
(45) Date of publication and mention of the grant of the patent: **31.07.2019**
(21) Application number: 10783193.5
(22) Date of filing: 09.03.2010
(51) Int. Cl.: G01N 21/78, A61B 5/1455, G01N 21/27

(54) **COMPONENT MEASURING DEVICE**
VORRICHTUNG ZUR KOMPONENTENMESSUNG
DISPOSITIF DE MESURE D'UN COMPOSANT

(30) Priority: 05.06.2009 JP 2009136654
(43) Date of publication of application: 11.04.2012
(73) Proprietor: Terumo Kabushiki Kaisha, Tokyo 151-0072 (JP); Hosiden Corporation, Yao-shi, Osaka 581-0071 (JP)
(72) Inventor: NISHIUCHI, Daisuke, Nakakoma-gun Yamanashi 409-3853 (JP); AIKAWA, Ryokei, Nakakoma-gun Yamanashi 409-3853 (JP); ASANO, Mitsuhiro, Yao-shi Osaka 581-0071 (JP); IWAKI, Toshikazu, Yao-shi Osaka 581-0071 (JP)
(74) Representative: TBK
(86) International application number: PCT/JP2010/053867
(87) International publication number: WO 2010/140407

(56) References cited:
- JP-A- 10 148 635
- JP-A- 63 101 757
- JP-A- 2000 262 298
- JP-A- 2000 329 707
- JP-A- 2005 091 315
- JP-A- 2007 093 273
- JP-A- 2008 020 446
- JP-A- 2008 051 822
- JP-T- 2001 522 043
- US-A- 5 611 999
- US-A- 6 055 060
- US-A1- 2003 214 655
- US-B1- 6 181 417
- US-B1- 6 201 607

## Description

The present invention relates to a component measuring device adapted to optically measure the level of the blood glucose in the blood, urinary components, or a specified component in a liquid such as drainage water, industrial water and the like according to the preamble of claim 1, the features of which are known from e.g. document US 6 201 607 B1.

Conventionally, a component measuring device for optically measuring a specified component has been used to detect chemical components in a body fluid or industrial water and measure the quantity and/or quality of the specified component. In the component measuring device, a test paper is impregnated with a liquid such as the blood, and then light having a predetermined wavelength is radiated onto the test paper. The specified component in the liquid-to-be-measured is calculated and measured by measuring the intensity of the light reflected at the test paper (see, for example, document JP H09-145615 A).

Further, document JP Hll-326192 A discloses an art in which two light sources having different wavelengths are provided for improving measurement accuracy, wherein the two light sources alternately radiate light onto the test paper.

In recent years, portable component measuring devices adapted to be carried by a user have been developed, and therefore miniaturization of the device is required. In order to miniaturize the device, it is considered to miniaturize the light source. Incidentally, due to increasing demand for devices provided with two light sources, such as the art described in Patent document 2, there is a strong demand for miniaturizing the light source. However, since a miniaturized light source can not have sufficient quantity of light necessary to accurately measure the component, it was difficult to miniaturize the light source and the device.

Further, since there is more or less individual variation in directionality of the light source, the diameter of the irradiation spot projected onto the test paper varies due to the individual variation in directionality of the light source. As a result, in a conventional component measuring device, since the diameter of the irradiation spot and illuminance distribution vary for every device, amount of data obtained by one device differs from amount of data obtained by another device, so that measurement error will be caused between different devices.

In view of the above problems, it is the object of the present invention to provide a component measuring device capable of efficiently radiating light onto the test paper even using a light source having low-light intensity, and improving the measurement accuracy by adjusting the diameter of the radiation spot projected onto test paper and the illuminance distribution.

The object of the invention is achieved by a component measuring device according to claim 1. Advantageous embodiments are carried out, according to the dependent claims. A component measuring device according to an aspect of the present invention includes a mounting portion on which a tip having a test paper attached thereto is mounted, the test paper being impregnated with a liquid, a light source adapted to emit light having a specified wavelength, and a light-receiving element adapted to receive the light reflected from the test paper. The component measuring device further includes a condensing lens where the light emitted from the light source enters and which condenses the light onto the test paper, and a light-path block arranged between the condensing lens and the light source, at the position of a photographic subject (object) of the condensing lens.

With the component measuring device of the present invention, by condensing light with the condensing lens, it is possible to efficiently radiate light onto the test paper even with small quantity of light. Thus, small light source having small light quantity can be used, so that it is possible to miniaturize the entire device. Further, by providing the diaphragm portion and setting the position of the photographic subject (object) of the condensing lens in the diaphragm portion, it is possible to adjust the diameter of the radiation spot projected onto the test paper. As a result, it is possible to obtain a predetermined irradiation shape and illuminance distribution regardless of individual variation of the light source, so that it is possible to improve the measurement accuracy and reduce measurement error between different devices.

FIG. 1 is a perspective view showing a component measuring device according to an embodiment of the present invention;
FIG. 2 is a right side view showing the component measuring device according to the aforesaid embodiment;
FIG. 3 is a front view showing the component measuring device according to the aforesaid embodiment;
FIG. 4 is a perspective view showing a primary portion of the component measuring device according to the present invention;
FIG. 5 is an exploded perspective view showing the primary portion of the component measuring device according to the present invention;
FIG. 6 is an enlarged cross section taken along line Y-Y of Fig. 3;
FIG. 7 is an enlarged cross section taken along line X-X of Fig. 2;
FIG. 8 is a perspective view showing a light-path block of the component measuring device according to the present invention;
FIG. 9 is an exploded perspective view showing the light-path block of the component measuring device according to the present invention; and
FIG. 10 is a graph showing illuminance distribution of the component measuring device according to the present invention which is provided with diaphragm portions, and illuminance distribution of a component measuring device which is not provided with the diaphragm portions.

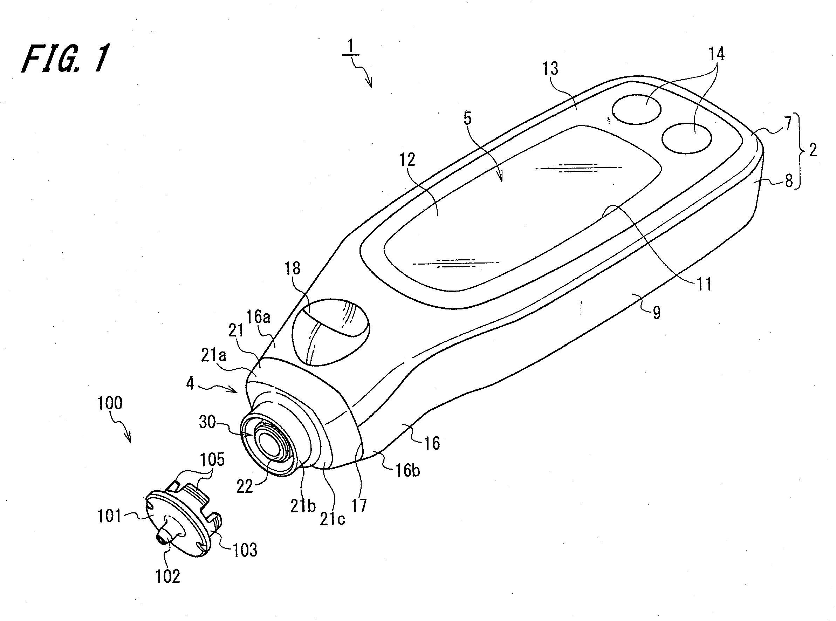

A component measuring device according to an embodiment of the present invention will be described below with reference to FIGS. 1 to 10. Note that, in the drawings, common components are denoted by same reference numerals. Further, it should be noted that the present invention is not limited to the following embodiment.

The description will be given in the following order.
1. Configuration example of component measuring device
2. Operation of component measuring device

### 1. Configuration example of component measuring device

The configuration of a blood glucose measuring device applied as the component measuring device according to an embodiment of the present invention (referred to as "present embodiment" hereinafter) will be described below with reference to FIGS. 1 to 3.

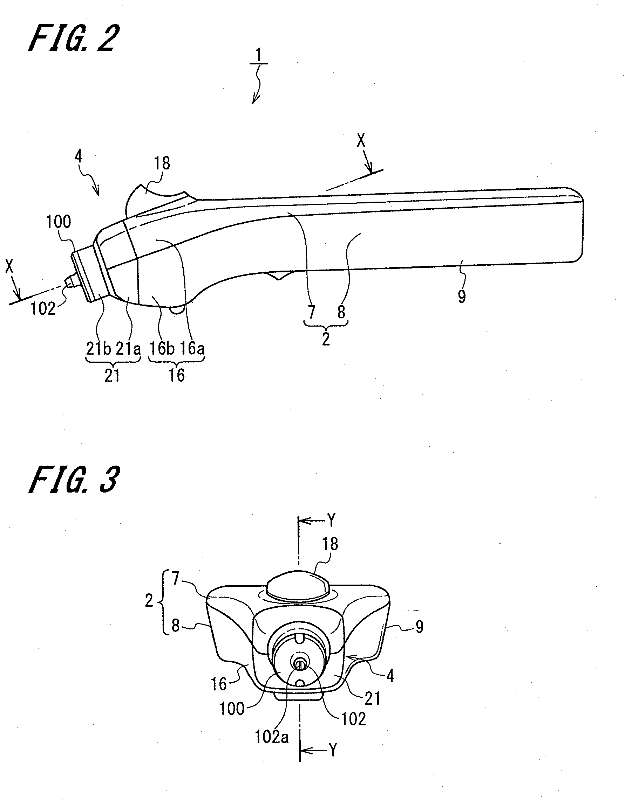

FIG. 1 is a perspective view of a blood glucose measuring device according to present embodiment, FIG. 2 is a right side view of the blood glucose measuring device, and FIG. 3 is a front view of the blood glucose measuring device.

The blood glucose measuring device 1 of the present embodiment is adapted to enable a doctor, a nurse, a diabetic patient or the like to collect blood in a tip, measure the level of the blood glucose in the blood, and manage the measured data of blood glucose level. The blood glucose measuring device can be operated on the palm of the hand of a user, just like a cellular phone.

As shown in FIG. 1, the blood glucose measuring device 1 includes a case 2 formed by a hollow container, a power section housed in the case 2, a measuring section 4 for measuring the blood glucose level and the like, a display 5, a controller for controlling the operation and display of the measuring section 4 and display 5, and the like. Further, a tip 100 for sucking and housing the blood of the user is mounted on the measuring section 4. Incidentally, input items, confirmation items, measurement result and the like, for example, are displayed on the display 5.

The case 2 is somewhat slender so that pressing operation of two operating switches 14 of an operating portion can be easily performed with holding it in one hand, and is formed into a three-dimensional shape allows a trunk portion 9 thereof to be fitted to the hand. The case 2 includes an upper case 7 and a lower case 8 superimposed on each other up and down. The case 2 is configured so as to be able to be assembled and disassembled by tightening and loosening a plurality of screws in a state where the upper case 7 and the lower case 8 are superimposed on each other up and down.

Further, the display 5, the controller and the like are arranged in the hollow portion of the trunk portion 9 of the case 2. Furthermore, the measuring section 4 is arranged on the side of a tip end (which is one end of the hollow portion in the longitudinal direction) of the case 2. The display 5 is attached to the upper case 7, and the power section is arranged in the lower case 8.

A battery housing portion is arranged in the lower case 8, wherein the battery housing portion opens on the lower surface of the lower case 8. For example, a button battery, as a portable power, is detachably housed in the battery housing portion. The battery housing portion is covered by a battery lid detachably attached to the lower case 8, and the battery housing portion can be opened and closed by the battery lid. In the blood glucose measuring device 1, the operation of the measuring section 4 and controller, and the display action of the display 5 are controlled by the power of the button battery.

The type of the battery used in the power section is not limited to the button battery, but includes other batteries such as round-shaped dry-cell battery, square-shaped dry-cell battery and the like. Further, on classifying according to material basis, an alkaline battery, a manganese dioxide battery and the like may obviously be used, and a secondary battery such as a lithium-ion battery may also be used.

A substantially rectangle-shaped opening window 11 is arranged substantially in the central portion of the upper case 7, the opening window 11 penetrating the front surface and the back surface of the upper case 7. A liquid crystal cover 12 is fitted in the opening window 11, the shape of the liquid crystal cover 12 matching the opening window 11. A liquid crystal panel of the display 5 is arranged on the back surface side of the liquid crystal cover 12. The entire upper face of the liquid crystal cover 12 is covered by a front panel 13, wherein the size of the front panel 13 is suitably larger than the size of the liquid crystal cover 12. The size of the front panel 13 is such that the front panel 13 covers the two operating switches 14 arranged on the upper face of the upper case 7.

The two operating switches 14 are respectively inserted into two through-holes formed in the upper face of the upper case 7. Various operations of the blood glucose measuring device 1, such as on/off operation and the like, can be performed through the operating switches 14.

The liquid crystal panel of the display 5 and a main wiring substrate of the controller are arranged between the upper case 7 and the lower case 8. The main wiring substrate is provided with an electrical circuit formed in a predetermined shape by printed wiring or the like. A microcomputer for executing a predetermined function previously designed, a storage device such as a ROM, a RAM or the like in which a predetermined program is previously stored, and other electronic components such as capacitor(s), resistor(s) and the like are mounted on the main wiring substrate.

As shown in FIG. 2, one side of the case 2 in the longitudinal direction is formed as a curved portion 16, which has a tapered shape and which slightly curves toward the side of the lower case 8. The curved portion 16 is formed by combining an upper curved portion 16a arranged in the upper case 7 and a lower curved portion 16b arranged in the lower case 8, wherein the upper curved portion 16a is an inward U-shaped portion, and the lower curved portion 16b is an outward U-shaped portion (see FIG. 3). The curved portion 16, which as a whole curves toward the side of the lower case 8, is formed by superimposing the upper curved portion 16a and the lower curved portion 16b on each other.

Further, an ejecting operator 18 is provided on the convex side of the curved portion 16. The ejecting operator 18 is a member for operating an ejecting member 24, which is to be described later. Incidentally, the curved portion 16 has a long hole formed therein for allowing the ejecting operator 18 to move. The long hole has a predetermined length linearly extending along the front-back direction of the case 2, and leg portion of the ejecting operator 18 is slidably engaged with the long hole.

Further, an opening 17 is provided at the tip end of the curved portion 16. The opening 17 opens obliquely downward at a position slightly leaning to the side of the lower case 8 from the portion passing through the center of the case 2. Incidentally, the measuring section 4 is arranged in the opening 17.

### [Measuring section]

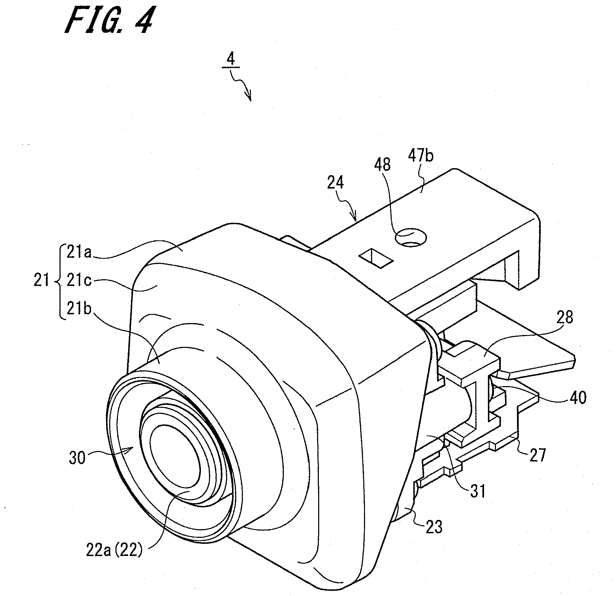

Next, the measuring section 4 of the present embodiment will be described below with reference to FIGS. 4 to 9.

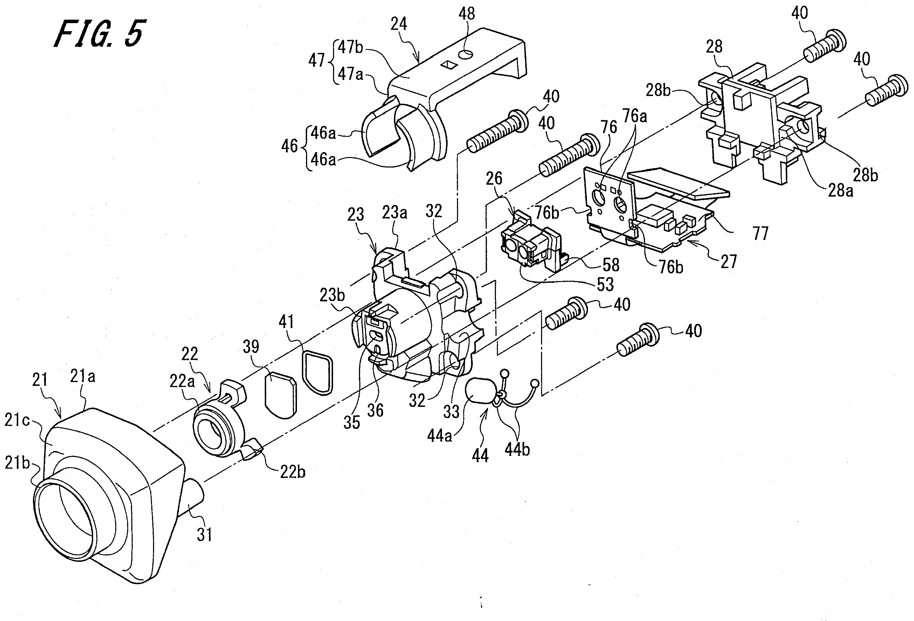

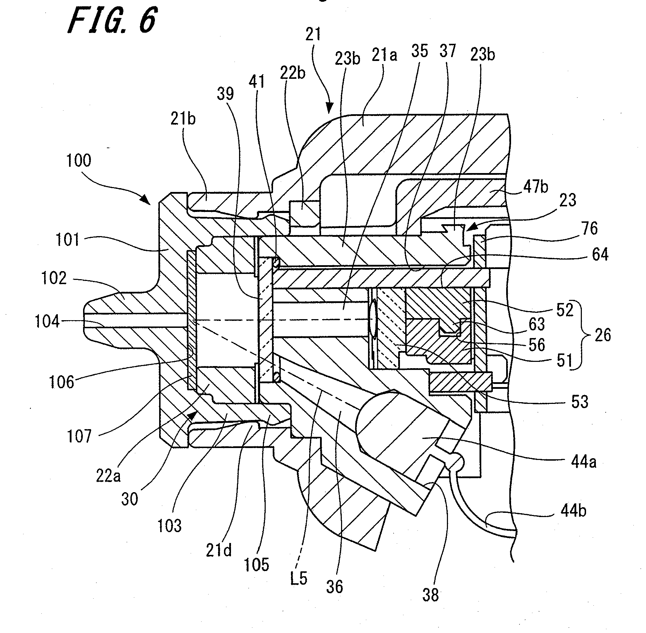

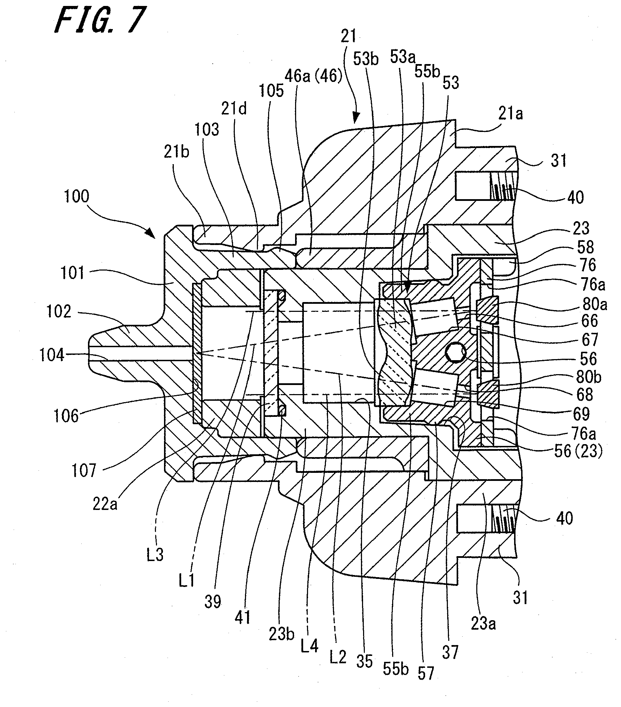

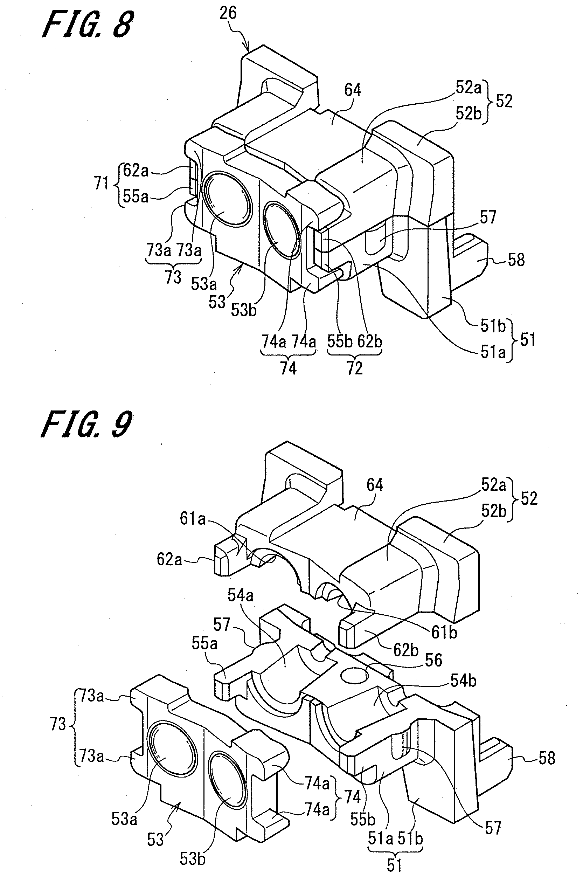

FIG. 4 is a perspective view of the measuring section of the present embodiment, FIG. 5 is an exploded perspective view of the measuring section, and FIG. 6 and FIG. 7 are each a cross section of the measuring section. Further, FIG. 8 is a perspective view of a light-path block of the measuring section of the present embodiment, and FIG. 9 is an exploded perspective view of the light-path block.

The measuring section 4 is a device for optically measuring blood glucose level by collecting the blood into the tip 100. The measuring section 4 includes a ring case 21, an inner ring 22, a photometric block 23, an ejecting member 24, a light-path block 26, a photometric substrate 27, and a substrate-pressing bracket 28 for pressing a light-emitting diode substrate.

As shown in FIG. 5, the ring case 21 has a fixing portion 21a for fixing the ring case 21 to the case 2, and a cylindrical shaft portion 21b integrally-formed continuously from the fixing portion 21a. The fixing portion 21a has an end face portion 21c which extends toward the center so that the opening 17 is closed. The cylindrical shaft portion 21b projects outward from substantially the central portion of the end face portion 21c. Further, screw portions 31, by which the photometric block 23 and the substrate-pressing bracket 28 are fixed through fixing screws 40, are arranged in the fixing portion 21a.

As shown in FIG. 6, a rib 21d is provided on the inner wall of the cylindrical shaft portion 21b. Further, the inner ring 22 is arranged on the inner side of the cylindrical shaft portion 21b. The inner ring 22 has a cylindrical portion 22a and a flange portion 22b. The flange portion 22b is formed continuously from the side of one end surface of the cylindrical portion 22a, and extends outwardly in the radial direction.

Further, the cylindrical portion 22a is arranged coaxially with the cylindrical shaft portion 21b on the inner side of the cylindrical shaft portion 21b of the ring case 21. Thus, a ring-like space portion is formed between the cylindrical shaft portion 21b of the ring case 21 and the cylindrical portion 22a of the inner ring 22. An ejecting portion 46 of the ejecting member 24 (which is to be described later) is slid in the longitudinal direction within the ring-like space portion (see FIG. 7). Further, a mounting portion 30 is configured by the ring case 21 and the inner ring 22, wherein the mounting portion 30 can support the tip 100 in a manner in which the tip 100 can be attached and detached. Incidentally, the photometric block 23 is arranged on the side of the back face of the inner ring 22 so as to face the inner ring 22.

As shown in FIG. 5, the photometric block 23 is formed as a hollow portion, and has a substantially plate-like base portion 23a and a substantially circular shaft portion 23b, wherein the shaft portion 23b is formed in front of the base portion 23a. The base portion 23a is provided with a plurality of screw holes 32 into which fixing screws are screwed, and two substantially semicircular notches 33. Incidentally, when installing the photometric block 23 onto the side of the back face of the ring case 21, the screw portions 31 of the ring case 21 is fitted into the two notches 33 (see FIG. 4).

As shown in FIG. 6, the shaft portion 23b is provided with a main light guide path 35 through which the light emitted from light-emitting diodes 80a, 80b (which are to be described later) is passed, and a light-receiving path 36 through which the light reflected by a test paper 107 attached to the tip 100 is passed. Incidentally, a housing portion 37, in which the light-path block 26 is housed, is arranged on an end portion of the main light guide path 35 on the side of the base portion 23a. The housing portion 37 has one face entirely opened on the side of the base portion 23a. Further, a fitting-in portion 38, into which a light-receiving element 44 is fitted, is provided on an end portion of the light-receiving path 36 on the side of the base portion 23a.

The light-receiving element 44 has a light-receiving section 44a and two wires 44b, wherein the light-receiving section 44a is adapted to receive the light reflected from the test paper 107. The light-receiving section 44a is formed in a cannon shell shape, and has a substantially spherical light-receiving surface. The two wires 44b extend continuously from the opposite side of the light-receiving surface of the light-receiving section 44a, and are electrically connected to the photometric substrate 27.

Further, a glass 39 is attached to an end surface of the shaft portion 23b through a rubber gasket 41 so that the opening of the main light guide path 35 and the opening of the light-receiving path 36 are closed.

By closing the opening of the main light guide path 35 and the opening of the light-receiving path 36 by the glass 39 and the rubber gasket 41, water and the like can be prevented from entering the photometric block 23.

Incidentally, the ejecting member 24 is slidably attached to the shaft portion 23b of the photometric block 23. The ejecting member 24 is provided for dismounting the tip 100 mounted on the mounting portion 30. The ejecting member 24 includes an ejecting portion 46, and a sliding plate 47 to which the ejecting portion 46 is fixed, wherein the ejecting portion 46 directly contacts with the tip 100 to eject the tip 100, and the sliding plate 47 is adapted to be slid with a predetermined distance.

The ejecting portion 46 is configured by a pair of arc-like pieces 46a, 46a facing each other, wherein the two arc-like pieces 46a, 46a are each curved in an arc shape. The ejecting portion 46 is attached to the shaft portion 23b of the photometric block 23 in a movable manner, and is adapted to be moved back and forth with a predetermined distance on the outer side of the shaft portion 23b along the axial direction.

The sliding plate 47 is formed in such a shape that the intermediate portion thereof in the longitudinal direction is bent by 90 degrees. The sliding plate has a fixing portion 47a arranged on one side of the bent portion thereof and has a connecting portion 47b arranged on the other side of the bent portion thereof. The fixing portion 47a is formed as a U-shaped portion whose size corresponds to the pair of the arc-like pieces 46a, 46a constituting the ejecting portion 46. The pair of arc-like pieces 46a, 46a erect from one surface of the fixing portion 47a so that they project toward the side opposite to the side of the connecting portion 47b. Further, a through-hole 48 for connecting the ejecting member 24 to the ejecting operator 18 is formed at substantially the central portion of the connecting portion 47b.

The ejecting member 24 is fixed to the ejecting operator 18 by tightening a fixing screw inserted into the through-hole 48 of the sliding plate 47. Further, the ejecting member 24 is pulled by a tension coil spring (not shown in the drawings) so as to be constantly biased backward in a direction to leave away from the inner ring 22.

Incidentally, by pressing the ejecting operator 18 forward, the ejecting member 24 is moved by a distance equivalent to the moving distance of the ejecting operator 18. As a result, a pair of tip ends of the ejecting portion 46 arranged on the tip end of the ejecting member 24 press the end portions of engagement claws 105 of the tip 100 forward, so that the tip 100 is dismounted from the mounting portion 30. By the aforesaid operation, the tip 100 can be discarded.

### [Tip]

Next, the tip 100 mounted on the blood glucose measuring device 1 will be described below. Referring back to Fig. 1, the tip 100 includes a disc-shaped base portion 101, a nozzle portion 102 formed on one surface of the base portion 101, and an engaging portion 103 formed on the other surface of the base portion 101.

The outer diameter of the base portion 101 is substantially equal to the outer diameter of the cylindrical shaft portion 21b. The nozzle portion 102 erects from the center of the base portion 101. A collecting hole 104 penetrating in the axial direction is provided in the central portion of the nozzle portion 102. The tip end portion of the nozzle portion 102 is tapered, and the tip end face of the tip end portion is provided with a recessed groove 102a for easily sucking the specimen (see FIG. 3).

The size of the engaging portion 103 of the tip 100 matches the space portion of the mounting portion 30. In other words, the outer diameter of the engaging portion 103 is set to such a value that allows the engaging portion 103 and the hole of the cylindrical shaft portion 21b to fit each other, and the inner diameter of the engaging portion 103 is set to such a value that allows the engaging portion 103 to cover the cylindrical portion 22a.

Further, four engagement claws 105 each formed by a resilient piece are provided in the tip end portion of the engaging portion 103. Incidentally, the number of the engagement claws 105 may also be three or less, or five or more. The engagement claws 105 are each formed as a circumferentially continuous arc-like convex portion, and, in order to increase frictional resistance, a plurality of convex portions are provided on the outer periphery of each of the engagement claws 105. The engagement claws 105 is brought into contact with the inner periphery of the cylindrical shaft portion 21b and pressed in, so that the engagement claws 105 cross over the rib 21d arranged in the inner face of the cylindrical shaft portion 21b, and thereby retention force for the cylindrical shaft portion 21b to retain the tip 100 is increased, which makes the tip 100 less likely to disengage (see FIG. 6).

Further, a test paper housing portion 106 in communication with the collecting hole 104 is provided in the inner side of the engaging portion 103. The test paper 107, which is adapted to collect the blood and hold a predetermined amount of blood, is housed in the test paper housing portion 106. The components of the blood can be measured by irradiating a specified light emitted from the photometric block 23 onto the test paper impregnated with the blood.

### [Light-path block]

Next, the light-path block 26 will be described below with reference to FIGS. 8 and 9. As shown in FIG. 8, the light-path block 26 includes a first light-path block 51 and a second light-path block 52. Further, a condensing lens 53 is attached to the light-path block 26.

As shown in FIG. 9, the first light-path block 51 has a base portion 51a and a flange portion 51b, wherein the base portion 51a has a substantially cuboid-shape. An end surface of the base portion 51a facing the condensing lens 53 is inclined from both ends in the longitudinal direction to the center of the base portion 51a, so that the central portion is recessed. The base portion 51a is provided with two grooves 54a, 54b each recessed in substantially circular shape, two convex pieces 55a, 55b, and an insertion hole 56.

A projection 57 is formed on each of both end surfaces of the base portion 51a in the longitudinal direction. As shown in FIG. 7, when housing the light-path block 26 into the housing portion 37 of the photometric block 23, the projections 57 abut the lateral wall of the housing portion 37, and therefore it is possible to position the light-path block 26 in the housing portion 37 of the photometric block 23 and prevent backlash of the light-path block 26.

The two grooves 54a, 54b are each recessed in two steps from one surface of the base portion 51a. The two grooves 54a, 54b are arranged substantially in a truncated V shape, and the insertion hole 56 is arranged between the two grooves 54a, 54b. The two convex pieces 55a, 55b approximately perpendicularly projects from the surface of the base portion 51a facing the condensing lens 53. The first convex piece 55a is formed on one end in the longitudinal direction, and the second convex piece 55b is formed on the other end in the longitudinal direction.

Further, the flange portion 51b is provided on a surface of the base portion 51a opposite to the surface facing the condensing lens 53. Two gripping pieces 58 for gripping the photometric substrate 27 are formed in the flange portion 51b.

The second light-path block 52 has the same configuration as that of the first light-path block 51, and has a base portion 52a and a flange portion 52b, wherein the base portion 52a has a substantially rectangular parallelepiped-shape. An end surface of the base portion 52a opposed to the condensing lens 53 is inclined from both ends in the longitudinal direction to the center of the base portion 52a, so that the central portion is recessed. The base portion 52a is provided with two grooves 61a, 61b each recessed in substantially circular shape, two convex pieces 62a, 62b, and a convex portion 63 (see FIG. 6) for being inserted into the insertion hole 56. Further, a mounting portion 64 recessed in substantially quadrangular shape is formed in the upper face portion of the base portion 52a.

The two grooves 61a, 61b are formed in a surface of the base portion 52a facing the two grooves 54a, 54b of the first light-path block 51. Similar to the two grooves 54a, 54b of the first light-path block 51, the two grooves 61a, 61b are each recessed in two steps. Incidentally, the two grooves 61a, 61b are arranged substantially in a truncated V shape so that the two grooves 61a, 61b face the two grooves 54a, 54b of the first light-path block 51. Further, the convex portion 63 projects between the two grooves 61a, 61b.

The two convex pieces 62a, 62b approximately perpendicularly projects from the surface of the base portion 52a facing the condensing lens 53. The first convex piece 62a is formed on one end in the longitudinal direction, and the second convex piece 62b is formed on the other end in the longitudinal direction. Further, the flange portion 52b is provided on a surface of the base portion 52a opposite to the surface facing the condensing lens 53.

A first diaphragm portion 66 and a first light guide path 67 are configured by the first groove 54a of the first light-path block 51 and the first groove 61a of the second light-path block 52. A second diaphragm portion 68 and a second light guide path 69 are configured by the second groove 54b of the first light-path block 51 and the second groove 61b of the second light-path block 52.

Further, a first fitting portion 71 is configured by the first convex piece 55a of the first light-path block 51 and the first convex piece 62a of the second light-path block 52. Further, a second fitting portion 72 is configured by the second convex piece 55b of the first light-path block 51 and the second convex piece 62b of the second light-path block 52.

The light emitted from light-emitting diodes 80a, 80b (which are to be described later) is incident on the condensing lens 53, and the condensing lens 53 condenses the light incident thereon onto the test paper 107 of the tip 100. The condensing lens 53 is formed in such a shape that it is substantially plate-like, and substantially the center thereof in the longitudinal direction is bent at an obtuse angle. Further, the condensing lens 53 includes a first lens portion 53a, a second lens portion 53b, and two fitting-receiving portions 73, 74.

The first lens portion 53a and the second lens portion 53b are each a convex lens which is convexed to both sides. The first lens portion 53a is formed on one side in the longitudinal direction, and the second lens portion 53b is formed on the other side in the longitudinal direction. When the condensing lens 53 has been attached to the light-path block 26, the first lens portion 53a is located in front of the first light guide path 67 and the first diaphragm portion 66. Further, the second lens portion 53b is located in front of the second light guide path 69 and the second diaphragm portion 68.

Incidentally, the focus of the first lens portion 53a on the incident side is set in front of the first diaphragm portion 66, and the focus of the second lens portion 53b on the incident side is set in front of the second diaphragm portion 68. The first diaphragm portion 66 and the second diaphragm portion 68 are respectively arranged at the position of the photographic subject (object) of the first lens portion 53a and the position of the photographic subject (object) of the second lens portion 53b. Further, as shown in FIG. 7, a first illumination axis L1 passing through the centers of both the first diaphragm portion 66 and the first lens portion 53a and a second illumination axis L2 passing through the centers of both the second diaphragm portion 68 and the second lens portion 53b intersect with each other at a point in the test paper 107 of the tip 100.

The first fitting-receiving portion 73 is arranged on one end of the condensing lens 53 in the longitudinal direction, and the second fitting-receiving portion 74 is arranged on the other end of the condensing lens 53 in the longitudinal direction. The first fitting-receiving portion 73 has two fitting pieces 73a, 73a arranged apart from each other by a predetermined distance. Further, as shown in FIG. 8, the first fitting portion 71 of the light-path block 26 is fitted between the two fitting pieces 73a, 73a of the first fitting-receiving portion 73. Similarly, the second fitting-receiving portion 74 has two fitting pieces 74a, 74a arranged apart from each other by a predetermined distance, and the second fitting portion 72 of the light-path block 26 is fitted between the two fitting pieces 74a, 74a.

Referring back to Fig. 5, the photometric substrate 27 and the substrate-pressing bracket 28 will be described below.

As shown in FIG. 5, the photometric substrate 27 includes an attachment portion 76 to which two light-emitting diodes 80a, 80b (which serve as light source) are attached, and a mounting portion 77 on which electronic components for controlling the light-emitting diodes 80a, 80b and the light-receiving element 44 are mounted. The attachment portion 76 is formed in a plate-shape and provided with two attachment holes 76a and two notches 76b formed therein.

As shown in FIG. 4, the photometric substrate 27 is supported by the substrate-pressing bracket 28 and arranged on the back side of the photometric block 23. Incidentally, as shown in FIG. 5, the substrate-pressing bracket 28 is provided with a supporting face portion 28a and a fixing hole 28b, wherein the supporting face portion 28a is adapted to press the photometric substrate 27 toward the side of the photometric block 23 and support the photometric substrate 27, and the fixing hole 28b is provided for fixing the substrate-pressing bracket 28 to the ring case 21 through the fixing screws 40.

Further, as shown in FIG. 7, the attachment portion 76 of the photometric substrate 27 is arranged so that the opening of the housing portion 37 of the photometric block 23 is closed. At this time, the first light-emitting diode 80a is arranged behind the first diaphragm portion 66 of the light-path block 26, and the second light-emitting diode 80b is arranged behind the second diaphragm portion 68 of the light-path block 26. Further, the gripping pieces 58 arranged in the light-path block 26 are engaged to the notches 76b of the attachment portion 76.

Incidentally, the wavelength of the first light-emitting diode 80a and the wavelength of the second light-emitting diode 80b are set different from each other. For example, the wavelength of the first light-emitting diode 80a is set to a range from 620nm to 640nm, which is utilized to detect glucose value obtained based on color density generated by the blood specimen. In contrast, the wavelength of the second light-emitting diode 80b is set to a range from 510nm to 540nm, which is utilized to detect hematocrit value obtained based on red-color density of the red blood cells.

The blood glucose measuring device 1 of the present embodiment is adapted to quantify the glucose concentration through correcting the glucose value by using the hematocrit value, to thereby measure the blood glucose level. Incidentally, the first light-emitting diode 80a and the second light-emitting diode 80b alternately emit light.

As shown in FIG. 7, the first illumination axis L1 passing through the centers of both the first diaphragm portion 66 and the first lens portion 53a is tilted with respect to the optical axis L3 of the first light-emitting diode 80a. Further, the second illumination axis L2 passing through the centers of both the second diaphragm portion 68 and the second lens portion 53b is tilted with respect to the optical axis L4 of the second light-emitting diode 80b. By providing the diaphragm portions 66, 68, it is not necessary to make the illumination axis L1 and the optical axis L3, as well as the illumination axis L2 and the optical axis L4 to respectively coincide with each other, so that the structure to which the light-emitting diodes 80a, 80b are attached is simplified.

Incidentally, the present embodiment is described based on an example in which two light sources are provided; however, the number of the light source may also be one, three, or more than three. Further, the number of the diaphragm portion and the number of the lens portions of the condensing lens are suitably set corresponding to the number of the light sources.

With the blood glucose measuring device 1 of the present embodiment, by condensing the light of the light-emitting diodes 80a, 80b by the condensing lens 53, it is possible to efficiently radiate light onto the test paper even with small quantity of light. As a result, since a small light-emitting diode with low-light intensity can be used, it is possible to miniaturize the entire device.

Further, owing to the condensing lens 53, it is possible to obtain sufficient quantity of light required for measurement even if the distance between the light-emitting diodes 80a, 80b and the test paper 107 (i.e., the length of the first and second light guide paths 67, 69 and the main light guide path 35) is increased. As a result, it makes possible to easily provide auxiliary functions, such as the tip ejecting mechanism such as the ejecting member 24, in the empty space.

### 2. Operation of component measuring device

The blood glucose measuring device 1 can be used in the following manner, for example. Incidentally, the tip 100 is previously mounted on the mounting portion 30 of the blood glucose measuring device 1. First, the blood of the user is collected by the tip 100. To be specific, a fingertip of the user is stuck by using a dedicated puncture device so that a slight amount (about 0.3 to 1.5µL, for example) of blood is flowed out from the stuck point onto the skin surface. The tip end of the nozzle portion 102 of the tip 100 mounted on the tip end of the blood glucose measuring device 1 is abutted to the lump of the blood flowed onto the fingertip.

By this operation, the blood on the fingertip enters the collecting hole 104 through the recessed groove 102a. The blood having entered the collecting hole 104 is sucked and flows in the inside thereof by the capillary action, so that the blood reaches the central portion of the test paper 107 housed in the engaging portion 103. The blood reached the test paper 107 infiltrates from the surface into the interior of the test paper 107, and spreads radially outward. While the blood is spreading, reaction between the glucose in the blood and chromogenic reagents carried on the test paper 107 is caused, so that the test paper 107 develops a color depending on the amount of the glucose.

Next, as shown in FIG. 7, the light of the first light-emitting diode 80a or the light of the second light-emitting diode 80b is radiated onto the test paper 107. In other words, the light emitted from the first light-emitting diode 80a passes through the first diaphragm portion 66 where the shape and diameter of the irradiation spot of the light is adjusted, and then the light passes through the first light guide path 67 to enter the first lens portion 53a of the condensing lens 53. Further, the light incident on the first lens portion 53a is condensed by the first lens portion 53a, and passes through the main light guide path 35 so as to be radiated onto the test paper 107.

Incidentally, the light emitted from the second light-emitting diode 80b passes through the second diaphragm portion 68 and the second light guide path 69 to enter the second lens portion 53b. Further, the light incident on the second lens portion 53b is condensed by the second lens portion 53a, and passes through the main light guide path 35 so as to be radiated onto the test paper 107.

Here, the first illumination axis L1 passing through the centers of both the first diaphragm portion 66 and the first lens portion 53a and the second illumination axis L2 passing through the centers of both the second diaphragm portion 68 and the second lens portion 53b intersect with each other at the test paper 107. Thus, the light emitted from the first light-emitting diode 80a and the light emitted from the second light-emitting diode 80b are both radiated at substantially the same point of the test paper 107.

Further, the position of the photographic subject (object) of the first lens portion 53a is set in the first diaphragm portion 66. Similarly, the position of the photographic subject (object) of the second lens portion 53b is set in the second diaphragm portion 68. Thus, the light narrowed by first diaphragm portion 66 or the second diaphragm portion 68 is projected onto the test paper 107. Thus, it is possible to adjust the shape and diameter of the irradiation spot projected onto the test paper 107 by adjusting the shape and diameter of both the first diaphragm portion 66 and the second diaphragm portion 68.

Since it is possible to adjust the shape and diameter of the irradiation spot projected onto the test paper 107, it is possible to reduce the diameter of the irradiation spot so as for the light-receiving element 44 to detect only the most necessary signal and reduce unnecessary noise. As a result, since the noise can be reduced, it is possible to improve the measurement accuracy.

Further, even there is some individual variation in directionality of the light-emitting diodes 80a, 80b, the position of the irradiation spot can be fixed by providing the diaphragm portions 66, 68. Thus, the measurement error between the different devices caused by individual variation of the directionality of the light-emitting diodes 80a, 80b can be suppressed to minimal level.

Next, as shown in FIG. 6, the light emitted from the first light-emitting diode 80a or the second light-emitting diode 80b is reflected by the test paper 107 and passes through the light-receiving path 36. Further, the light L5 having passed through the light-receiving path 36 reaches the light-receiving element 44 where the quantity of light is measured. Thereby it is possible to measure the color of the test paper 107 so as to measure the degree of coloration, so that the blood glucose level can be obtained.

In the case where the tip 100 is to be ejected from the mounting portion 30 after measurement, the user may, for example, grasp the trunk portion 9 and put his (or her) thumb on the depression of the ejecting operator 18. Further, the user may press the ejecting operator 18 toward the front side to slide the ejecting member 24 forward. At this time, from the state of FIGS. 2 and 3, when pressing the ejecting operator 18 forward, the ejecting member 24 integral with the ejecting operator 18 will be moved by the same distance. As a result, as shown in FIG. 7, a pair of tip ends of the ejecting portion 46 arranged on the tip end of the ejecting member 24 will press the end portion of the engagement claw 105 of the tip 100 forward, so that the tip 100 is dismounted from the mounting portion 30. Thus, it is possible to discard the tip 100 into a waste container or the like.

In such case, in the state where the blood glucose measuring device 1 has been held by one hand, the user who operates the blood glucose measuring device 1 can easily separate the tip 100 from the blood glucose measuring device 1 and discard the tip 100 by one-handed operation. Further, the case 2 is provided with the curved portion 16, and the ejecting operator 18 is arranged on the convex side of the curved portion 16. Furthermore, since the mounting portion 30 is arranged on the tip end of the curved portion 16, the tip 100 is expelled in the moving direction of the ejecting operator 18. Thus, it is possible to aim a desired ejecting direction, and therefore it is possible to eject the tip 100 into the waste container with simplicity and reliability. Thus, it is possible to dispose the tip 100 in a safe, simple and quick manner without touching the tip 100 by hand.

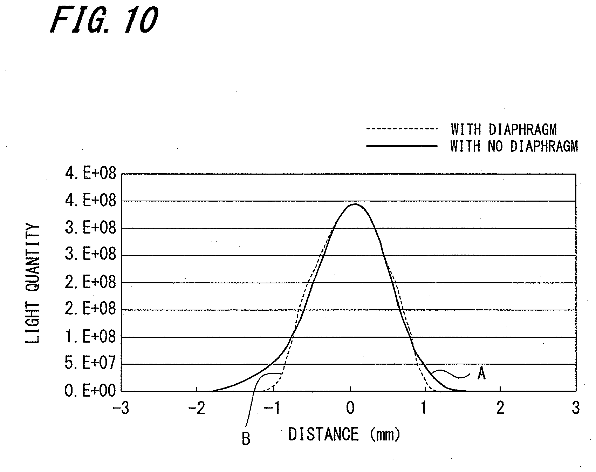

Next, illuminance distribution of the light radiated onto the test paper of the blood glucose measuring device 1 of the present embodiment which is provided with the diaphragm portions and illuminance distribution of the light radiated onto the test paper of a blood glucose measuring device which is not provided with the diaphragm portions will be described below with reference to FIG. 10.

FIG. 10 is a graph showing the illuminance distribution of the blood glucose measuring device according to the present embodiment and the illuminance distribution of a blood glucose measuring device having no diaphragm portion. In FIG. 10, the vertical axis represents light quantity, and the horizontal axis represents the distance (mm) from the center of the irradiation spot projected onto the test paper 107.

It is known from FIG. 10 that, in the illuminance of the blood glucose measuring device having no diaphragm indicated by solid line A, the irradiation spot of light spreads to the points 2 mm away from the center. Thus, amount of data detected by the light-receiving element 44 becomes large, so that even unnecessary noise has been detected. Further, the more different of the graph shape of the illuminance distribution from a rectangle, the larger difference in spatial resolution of the light will be caused. As a result, in a conventional blood glucose measuring device having no diaphragm, since a large amount of noise has been detected and since difference in measurement area becomes large, the measurement accuracy is impaired.

In contrast, it is known from FIG. 10 that, in the illuminance of the blood glucose measuring device of the present embodiment provided with the diaphragm portions indicated by broken line B, the diameter of the irradiation spot is smaller and the shape of the illuminance distribution is more close to a rectangle compared with the blood glucose measuring device provided with no diaphragm portion. Thus, unnecessary increasing of the amount of data detected by the light-receiving element 44 is suppressed, so that difference in spatial resolution can be suppressed. As a result, it is possible to reduce noise detected by the light-receiving element 44; and further, since the difference in measurement area can be reduced, it is possible to improve the measurement accuracy.

It is to be understood that the present invention is not limited to the embodiment described above and shown in the attached drawings, and various modifications can be made without departing from the scope of the present invention.

Incidentally, although the aforesaid embodiment is described based on an example in which the liquid-to-be-measured is blood, the present invention is not limited such example. For example, the liquid-to-be-measured may also be a body fluid other than blood such as lymph, spinal fluid, saliva or the like; or various other liquids such as drainage water, industrial water or the like.

Further, although the aforesaid embodiment is described based on an example in which the target component to be measured in the body fluid (the blood) is glucose (blood glucose level), the present invention is not limited such example. For example, the target component to be measured may also be cholesterol, uric acid, creatinine, lactic acid, hemoglobin (occult blood), various alcohols, various saccharides, various proteins, various vitamins, various inorganic ions such as sodium ion and the like, and environmental hormones (endocrine disrupters) such as PCB, dioxin and the like. Furthermore, although the aforesaid embodiment is described based on an example in which the quantity of the specified component is measured, the present invention may also be applied to measure the quality of the specified component, or measure both the quantity and the quality of the specified component.

Incidentally, although the aforesaid embodiment is described based on an example in which the ejecting portion of the ejecting member is configured by two arc-like portions facing each other, the ejecting portion of the ejecting member may also configured by a combination of three or more portions. Further, the shape of these portions may also have a U-shape, a L-shape, instead of having an arc-like shape.

Further, although the aforesaid embodiment is described based on an example in which the two light-emitting diodes used as the light source are arranged in parallel on the same surface of the substantially plate-like substrate, the two light-emitting diodes may also each be arranged at a tilt so that the optical axis and the illumination axis thereof coincide with each other. Incidentally, although the aforesaid embodiment is described based on an example in which the light-emitting diodes (LED) are used as the light source, various other light emitters such as laser diode or the like may also be used as the light source.

### List of Reference Numerals

1 blood glucose measuring device (component measuring device)
2 case
4 measuring section
7 upper case
8 lower case
9 trunk portion
18 ejecting operator
21 ring case
21a fixing portion
21b cylindrical shaft portion
21c end face portion
21d rib
22 inner ring
22a cylindrical portion
23 photometric block
24 ejecting member
26 light-path block
27 photometric substrate
28 substrate-pressing bracket
30 mounting portion
35 main light guide path
36 light-receiving path
37 housing portion
38 fitting-in portion
39 glass
41 rubber gasket
44 light-receiving element
44a light-receiving section
44b wire
46 ejecting portion
51 first light-path block
51a, 51a base portions
51b, 51b flange portions
52 second light-path block
53 condensing lens
53a first lens portion
53b second lens portion
66 first diaphragm portion
67 first light guide path
68 second diaphragm portion
69 second light guide path
71 first fitting portion
72 second fitting portion
73 first fitting-receiving portion
74 second fitting-receiving portion
76 attachment portion
76a attachment holes
80a first light-emitting diode (light source)
80b second light-emitting diode (light source)
100 tip
104 collecting hole
107 test paper
L1 first illumination axis
L2 second illumination axis
L3, L4 optical axes

## Claims

1. A component measuring device comprising:
a mounting portion (30) on which a tip (100) having a test paper (107) attached thereto is mounted, the test paper (107) being impregnated with a liquid;
a light source (80a, 80b) adapted to emit light having a specified wavelength;
a condensing lens (53) where the light emitted from the light source (80a, 80b) enters and which condenses the light on the test paper (107);
a light-path block (26) including a diaphram portion (66, 68) arranged between the condensing lens (53) and the light source (80a, 80b), at the position of a photographic subject (object) of the condensing lens; and
a light-receiving element (44a) adapted to receive the light reflected from the test paper (107)
**characterized in that**
an illumination axis (L1) passing through the center of the diaphragm portion (66) and the center of the condensing lens (53) is tilted with respect to an optical axis (L3) of the light source (80a, 80b).

2. The component measuring device according to claim 1,
wherein the light source is configured by a first light source (80a) having a first wavelength, and a second light source (80b) having a second wavelength different from the first wavelength,
wherein the diaphragm portion includes a first diaphragm portion (66) through which the light from the first light source (80a) is passed, and a second diaphragm portion (68) through which the light from the second light source (80b) is passed, and
wherein the condensing lens (53) includes a first condensing lens (53a) through which the light from the first light source (80a) is transmitted, and a second condensing lens (53b) through which the light from the second light source (80b) is transmitted.

3. The component measuring device according to claim 2, wherein the light emitted from the first light source (80a) and the light emitted from the second light source (80b) are radiated onto substantially the same position on the test paper (107).

4. The component measuring device according to claim 2 or 3,
wherein the first light source (80a) and the second light source (80b) are arranged in parallel on the same surface of a substantially plate-like substrate,
wherein a first illumination axis (L1) passing through the centers of both the first diaphragm portion (66) and the first lens portion (53a) is tilted with respect to a first optical axis (L3) of the first light source (80a), and
wherein a second illumination axis (L2) passing through the centers of both the second diaphragm portion (68) and the second lens portion (53b) is tilted with respect to a second optical axis (L4) of the second light source (80b).

5. The component measuring device according to any one of claims 1 to 4, wherein the condensing lens (53) is integrally attached to the light-path block (26).

## Patentansprüche

1. Komponentenmessvorrichtung mit:
einem Montageabschnitt (30), an den eine Spitze (100) montiert ist, die ein Indikatorpapier (107) daran angebracht aufweist, wobei das Indikatorpapier (107) mit einer Flüssigkeit gedrängt ist;
einer Lichtquelle (80a, 80b), die angepasst ist, ein Licht abzugeben, das eine bestimmte Wellenlänge aufweist;
einer Kondensatorlinse (53), in die das von der Lichtquelle (80a, 80b) abgegebene Licht eintritt, und die das Licht auf dem Indikatorpapier (107) verdichtet;
einem Lichtpfadblock (26) mit einem Membranabschnitt (66, 68), der zwischen der Kondensatorlinse (53) und der Lichtquelle (80a, 80b) an der Position eines fotografischen Gegenstands (Objekt) der Kondensatorlinse angeordnet ist; und
einem Lichtempfangselement (44a), das angepasst ist, das von dem Indikatorpapier (107) reflektierte Licht zu empfangen,
**dadurch gekennzeichnet, dass**
eine Beleuchtungsachse (L1), die durch die Mitte des Membranabschnitts (66) und die Mitte der Kondensatorlinse (53) durchgeht mit Bezug auf eine optische Achse (L3) der Lichtquelle (80a, 80b) gekippt ist.

2. Komponentenmessvorrichtung nach Anspruch 1,
wobei die Lichtquelle durch eine erste Lichtquelle (80a) mit einer ersten Wellenlänge und eine zweite Lichtquelle (80b) mit einer zweiten Wellenlänge, die unterschiedlich zu der ersten Wellenlänge ist, konfiguriert ist,
wobei der Membranabschnitt einen ersten Membranabschnitt (66) hat, durch den das Licht von der ersten Lichtquelle (80a) durchgeschickt wird, und einen zweiten Membranabschnitt (68), durch den das Licht von der zweiten Lichtquelle (80b) durchgeschickt wird, und
wobei die Kondensatorlinse (53) eine erste Kondensatorlinse (53a) hat, durch die das Licht von der ersten Lichtquelle (80a) übertragen wird, und eine zweite Kondensatorlinse (53b), durch die das Licht von der zweiten Lichtquelle (80b) übertragen wird.

3. Komponentenmessvorrichtung nach Anspruch 2, wobei das von der ersten Lichtquelle (80a) und das von der zweiten Lichtquelle (80b) abgegebene Licht auf im Wesentlichen die gleiche Position auf dem Indikatorpapier (107) abgestrahlt werden.

4. Komponentenmessvorrichtung nach Anspruch 2 oder 3,
wobei die erste Lichtquelle (80a) und die zweite Lichtquelle (80b) parallel auf der gleichen Oberfläche eines im Wesentlichen scheibenartigen Substrats angeordnet sind,
wobei eine erste Beleuchtungsachse (L1), die durch die Mitten von sowohl dem ersten Membranabschnitt (66) wie auch des ersten Linsenabschnitts (53a) durchgeht, mit Bezug auf eine erste optische Achse (L3) der ersten Lichtquelle (80a) gekippt ist, und
wobei eine zweite Beleuchtungsachse (L2), die durch die Mitten von sowohl dem zweiten Membranabschnitt (68) wie auch dem zweiten Linsenabschnitt (53b) durchgeht, mit Bezug auf eine zweite optische Achse (L4) der zweiten Lichtquelle (80a) gekippt ist.

5. Komponentenmessvorrichtung nach einem der Ansprüche 1 bis 4, wobei die Kondensatorlinse (53) einstückig an den Lichtpfadblock (26) angebracht ist.

## Revendications

1. Dispositif de mesure de composé comprenant :
une partie de montage (30) sur laquelle est montée une pointe (100) ayant un papier réactif (107) fixé sur celle-ci, le papier réactif (107) étant imprégné d'un liquide ;
une source de lumière (80a, 80b) adaptée pour émettre une lumière ayant une longueur d'onde spécifiée ;
une lentille de condensation (53) où pénètre la lumière émise depuis la source de lumière (80a, 80b) et qui condense la lumière sur le papier réactif (107) ;
un bloc de chemin lumineux (26) comportant une partie de diaphragme (66, 68) agencée entre la lentille de condensation (53) et la source de lumière (80a, 80b), à la position d'un sujet photographique (objet) de la lentille de condensation ; et
un élément récepteur de lumière (44a) adapté pour recevoir la lumière réfléchie depuis le papier réactif (107) **caractérisé en ce que**
un axe d'éclairage (L1) traversant le centre de la partie de diaphragme (66) et le centre de la lentille de condensation (53) est incliné par rapport à un axe optique (L3) de la source de lumière (80a, 80b).

2. Dispositif de mesure de composé selon la revendication 1,
dans lequel la source de lumière est configurée par une première source de lumière (80a) ayant une première longueur d'onde, et une seconde source de lumière (80b) ayant une seconde longueur d'onde différente de la première longueur d'onde,
dans lequel la partie de diaphragme comporte une première partie de diaphragme (66) à travers laquelle passe la lumière provenant de la première source de lumière (80a), et
une seconde partie de diaphragme (68) à travers laquelle passe la lumière provenant de la seconde source de lumière (80b), et
dans lequel la lentille de condensation (53) comporte une première lentille de condensation (53a) à travers laquelle est transmise la lumière provenant de la première source de lumière (80a), et une seconde lentille de condensation (53b) à travers laquelle est transmise la lumière provenant de la seconde source de lumière (80b).

3. Dispositif de mesure de composé selon la revendication 2, dans lequel la lumière émise depuis la première source de lumière (80a) et la lumière émise depuis la seconde source de lumière (80b) sont rayonnées sur sensiblement la même position sur le papier réactif (107).

4. Dispositif de mesure de composé selon la revendication 2 ou 3,
dans lequel la première source de lumière (80a) et la seconde source de lumière (80b) sont agencées en parallèle sur la même surface d'un substrat sensiblement en forme de plaque,
dans lequel un premier axe d'éclairage (L1) traversant à la fois les centres de la première partie de diaphragme (66) et de la première partie de lentille (53a) est incliné par rapport à un premier axe optique (L3) de la première source de lumière (80a), et
dans lequel un second axe d'éclairage (L2) traversant à la fois les centres de la seconde partie de diaphragme (68) et de la seconde partie de lentille (53b) est incliné par rapport à un second axe optique (L4) de la seconde source de lumière (80b).

5. Dispositif de mesure de composé selon l'une quelconque des revendications 1 à 4, dans lequel la lentille de condensation (53) est fixée d'un seul tenant sur le bloc de chemin lumineux (26).
